# EUROPEAN PATENT SPECIFICATION

(11) **EP 2 709 976 B1**
(45) Date of publication and mention of the grant of the patent: **25.03.2015**
(21) Application number: 12735710.1
(22) Date of filing: 28.06.2012
(51) Int. Cl.: C07C 43/205

(54) **BIPHENOL ETHER COMPOUNDS**
BIPHENOLETHERVERBINDUNGEN
COMPOSÉS D'ÉTHER DE BISPHÉNOL

(30) Priority: 30.06.2011 US 201161502973 P
(43) Date of publication of application: 26.03.2014
(73) Proprietor: Dow Global Technologies LLC, Midland, MI 48674 (US)
(72) Inventor: GREEN, George D., Cary, IL 60013 (US); SWEDO, Raymond, Mount Prospect, IL 60056 (US)
(74) Representative: Buckley, Guy Julian
(86) International application number: PCT/US2012/044551
(87) International publication number: WO 2013/003538

(56) References cited:
- US-A1- 2004 250 469
- US-A1- 2005 042 195
- ALLAN B. GAMBLE ET AL: "Synthesis of reaction-ready 6,6'-biindole and 6,6'-biisatin via palladium(ii)-catalysed intramolecular C-H functionalisation", CHEMICAL COMMUNICATIONS, vol. 46, no. 23, 1 January 2010 (2010-01-01), page 4076, XP55040976, ISSN: 1359-7345, DOI: 10.1039/c002098b
- ALEXANDRE ALEXAKIS ET AL: "Biphenol-Based Phosphoramidite Ligands for the Enantioselective Copper-Catalyzed Conjugate Addition of Diethylzinc", THE JOURNAL OF ORGANIC CHEMISTRY, vol. 69, no. 17, 1 August 2004 (2004-08-01), pages 5660-5667, XP55027334, ISSN: 0022-3263, DOI: 10.1021/jo049359m
- ANGELOVSKI G ET AL: "Synthesis of hydroquinone-, biphenol-, and binaphthol-containing aza macroheterocycles via regioselective hydroformylation and reductive amination", TETRAHEDRON, ELSEVIER SCIENCE PUBLISHERS, AMSTERDAM, NL, vol. 59, no. 41, 6 October 2003 (2003-10-06), pages 8265-8274, XP004458583, ISSN: 0040-4020, DOI: 10.1016/J.TET.2003.08.012
- SHUANG-QUAN ZANG ET AL: "Diverse Intermolecular Interactions in Metal-Organic Frameworks Constructed with the New Supramolecular Synthon Ag n -L-Ag n ( n = 4, 5) (H 2 L = 2,2'-Bis(prop-2-ynyloxy)biphenyl)", ORGANOMETALLICS, vol. 27, no. 11, 1 June 2008 (2008-06-01), pages 2396-2398, XP55040984, ISSN: 0276-7333, DOI: 10.1021/om800165x
- DHANALEKSHMI S ET AL: "Anodic oxidation of aryl propargyl and aryl allyl ethers at a platinum electrode", TETRAHEDRON, ELSEVIER SCIENCE PUBLISHERS, AMSTERDAM, NL, vol. 50, no. 21, 1 January 1994 (1994-01-01), pages 6387-6400, XP026648681, ISSN: 0040-4020, DOI: 10.1016/S0040-4020(01)80655-4 [retrieved on 1994-01-01]
- PRASTARO A ET AL: "Homocoupling of arylboronic acids and potassium aryltrifluoroborates catalyzed by protein-stabilized palladium nanoparticles under air in water", TETRAHEDRON LETTERS, ELSEVIER, AMSTERDAM, NL, vol. 51, no. 18, 5 May 2010 (2010-05-05), pages 2550-2552, XP027077193, ISSN: 0040-4039, DOI: 10.1016/J.TETLET.2010.03.015 [retrieved on 2010-03-11]
- SHRIYA H. WADUMETHRIGE ET AL: "A Facile Synthesis of Elusive Alkoxy-Substituted Hexa- peri- hexabenzocoronene", ORGANIC LETTERS, vol. 10, no. 22, 20 November 2008 (2008-11-20), pages 5139-5142, XP55041002, ISSN: 1523-7060, DOI: 10.1021/ol8020429

## Description

This invention relates to compounds useful in a method for marking liquid hydrocarbons and other fuels and oils.

Marking of petroleum hydrocarbons and other fuels and oils with various kinds of chemical markers is well known in the art. A variety of compounds have been used for this purpose, as well as numerous techniques for detection of the markers, e.g., absorption spectroscopy and mass spectrometry. For example, U.S. Pat. No. 7,858,373 discloses the use of a variety of organic compounds for use in marking liquid hydrocarbons and other fuels and oils. US 2004/250469 discloses a method for marking hydrocarbons with substituted anthraquinones.

However, there is always a need for additional marker compounds for these products. Combinations of markers can be used as digital marking systems, with the ratios of amounts forming a code for the marked product. Additional compounds useful as fuel and lubricant markers would be desirable to maximize the available codes. The problem addressed by this invention is to find additional markers useful for marking liquid hydrocarbons and other fuels and oils.

### STATEMENT OF INVENTION

The present invention is as set out in the accompanying claims.

The present invention provides a compound having formula (I), 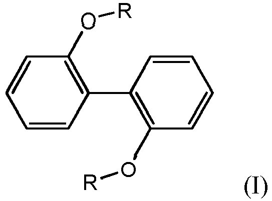 wherein R represents C₁₀-C₁₈ alkyl or C₅-C₁₈ alkenyl.

### DETAILED DESCRIPTION

Percentages are weight percentages (wt%) and temperatures are in °C, unless specified otherwise. Concentrations are expressed either in parts per million ("ppm") calculated on a weight/weight basis, or on a weight/volume basis (mg/L); preferably on a weight/volume basis. The term "petroleum hydrocarbon" refers to products having a predominantly hydrocarbon composition, although they may contain minor amounts of oxygen, nitrogen, sulfur or phosphorus; petroleum hydrocarbons include crude oils as well as products derived from petroleum refining processes; they include, for example, crude oil, lubricating oil, hydraulic fluid, brake fluid, gasoline, diesel fuel, kerosene, jet fuel and heating oil. Marker compounds of this invention can be added to a petroleum hydrocarbon or a liquid biologically derived fuel; examples of the latter are biodiesel fuel, ethanol, butanol, ethyl tert-butyl ether or mixtures thereof. A substance is considered a liquid if it is in the liquid state at 20°C. A biodiesel fuel is a biologically derived fuel containing a mixture of fatty acid alkyl esters, especially methyl esters. Biodiesel fuel typically is produced by transesterification of either virgin or recycled vegetable oils, although animal fats may also be used. An ethanol fuel is any fuel containing ethanol, in pure form, or mixed with petroleum hydrocarbons, e.g., "gasohol." An "alkyl" group is a substituted or unsubstituted hydrocarbyl group having from one to eighteen carbon atoms which may be in a linear or branched arrangement. An "alkenyl" group is an alkyl group containing one or more double bonds. Substitution on alkyl groups of one or more hydroxy or alkoxy groups is permitted. Preferably, alkyl groups are unsubstituted. Preferably, alkyl groups are acyclic. Preferably, the compounds of this invention contain elements in their naturally occurring isotopic proportions.

R represents C₁₀-C₁₈ alkyl or C₅-C₁₈ alkenyl; preferably C₁₀-C₁₈ alkyl; preferably C₁₀-C₁₆ alkyl. In formula (I), the -OR groups are in the 2,2'-positions on the benzene rings, i.e., the compounds have the following structure:

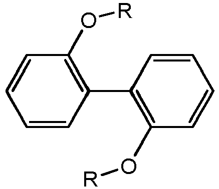

Preferably the minimum amount of each marker added to a petroleum hydrocarbon, a biodiesel fuel, an ethanol fuel, or a mixture thereof is at least 0.01 ppm, preferably at least 0.02 ppm, preferably at least 0.05 ppm, preferably at least 0.1 ppm, preferably at least 0.2 ppm. Preferably, the maximum amount of each marker is 50 ppm, preferably 20 ppm, preferably 15 ppm, preferably 10 ppm, preferably 5 ppm, preferably 2 ppm, preferably 1 ppm, preferably 0.5 ppm. Preferably, the maximum total amount of marker compounds is 100 ppm, preferably 70 ppm, preferably 50 ppm, preferably 30 ppm, preferably 20 ppm, preferably 15 ppm, preferably 12 ppm, preferably 10 ppm, preferably 8 ppm, preferably 6 ppm, preferably 4 ppm, preferably 3 ppm, preferably 2 ppm, preferably 1 ppm. Preferably, a marker compound is not detectible by visual means in the marked petroleum hydrocarbon or liquid biologically derived fuel, i.e., it is not possible to determine by unaided visual observation of color or other characteristics that it contains a marker compound. Preferably, a marker compound is one that does not occur normally in the petroleum hydrocarbon or liquid biologically derived fuel to which it is added, either as a constituent of the petroleum hydrocarbon or liquid biologically derived fuel itself, or as an additive used therein.

Preferably, the marker compounds have a log P value of at least 3, where P is the 1-octanol/water partition coefficient. Preferably, the marker compounds have a log P of at least 4, preferably at least 5. Log P values which have not been experimentally determined and reported in the literature can be estimated using the method disclosed in Meylan, W.M & Howard, P.H., J. Pharm. Sci., vol. 84, pp. 83-92 (1995). Preferably the petroleum hydrocarbon or liquid biologically derived fuel is a petroleum hydrocarbon, biodiesel fuel or ethanol fuel; preferably a petroleum hydrocarbon or biodiesel fuel; preferably a petroleum hydrocarbon; preferably crude oil, gasoline, diesel fuel, kerosene, jet fuel or heating oil; preferably gasoline.

Preferably, the marker compounds are detected by at least partially separating them from constituents of the petroleum hydrocarbon or liquid biologically derived fuel using a chromatographic technique, e.g., gas chromatography, liquid chromatography, thin-layer chromatography, paper chromatography, adsorption chromatography, affinity chromatography, capillary electrophoresis, ion exchange and molecular exclusion chromatography. Chromatography is followed by at least one of: (i) mass spectral analysis, and (ii) FTIR. Identities of the marker compounds preferably are determined by mass spectral analysis. Preferably, mass spectral analysis is used to detect the marker compounds in the petroleum hydrocarbon or liquid biologically derived fuel without performing any separation. Alternatively, marker compounds may be concentrated prior to analysis, e.g., by distilling some of the more volatile components of a petroleum hydrocarbon or liquid biologically derived fuel.

Preferably, more than one marker compound is present. Use of multiple marker compounds facilitates incorporation into the petroleum hydrocarbon or liquid biologically derived fuel of coded information that may be used to identify the origin and other characteristics of the petroleum hydrocarbon or liquid biologically derived fuel. The code comprises the identities and relative amounts, e.g., fixed integer ratios, of the marker compounds. One, two, three or more marker compounds may be used to form the code. Marker compounds according to this invention may be combined with markers of other types, e.g., markers detected by absorption spectrometry, including those disclosed in U.S. Pat. No. 6,811,575; U.S. Pat. App. Pub. No. 2004/0250469 and EP App. Pub. No. 1,479,749. Marker compounds are placed in the petroleum hydrocarbon or liquid biologically derived fuel directly, or alternatively, placed in an additives package containing other compounds, e.g., antiwear additives for lubricants, detergents for gasoline, etc., and the additives package is added to the petroleum hydrocarbon or liquid biologically derived fuel.

The compounds of this invention may be prepared by methods known in the art. For example, alkyl halides may react with biphenols in the presence of base according to the following equation

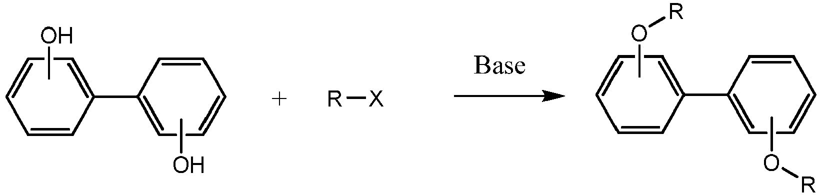

### EXAMPLES

The synthesis of biphenol ethers is illustrated by the following example:
2,2'-Bis(dodecyloxy)-1,1'-biphenyl: A 100 mL 3-neck flask was equipped with a magnetic stirrer, a reflux condenser with nitrogen blanket, and a heating mantle with a temperature controller and a thermocouple. The flask was charged with 3.74 grams (0.02 moles) of 2,2'-biphenol, 2.8 grams (0.04 moles, 85 wt. %) of potassium hydroxide, and with 25 mL of dimethylsulfoxide. The mixture was stirred under nitrogen while heating to 100°C. After about 2½ hours, the potassium hydroxide had dissolved, and the mixture was cooled to about 70°C. Dodecyl bromide (9.60 mL; d 1.038; 9.97 grams; 0.04 moles) was added in one portion. An exotherm to about 86°C was observed. After the exotherm subsided, the reaction mixture was stirred at 70°C. After about 5 hours, the reaction mixture was poured into about 400 mL of water. The white solids that separated were collected by filtration, and were washed on the filter with several portions of water. The solids were first air-dried, and then were dried in a vacuum oven at 50°C for about 2 hours. The yield of product was 9.49 grams (91%), having a melting point of 33 - 35°C. The structure was confirmed by IR, ¹H- and ¹³C-NMR, and GC/MS analyses.

In those cases in which, upon quenching the reaction mixture in water, the product separated out as an oil, extraction with ethyl ether was used in place of filtration.

### 2,2'-Biphenol Ethers Prepared:

| R | % Yield | MP,°C |
|---|---|---|
| | | |
| n-C₈H₁₇ (BOct-BBPh) | 94 | (oil) |
| n-C₁₀H₂₁ (BDec-BBPh) | 97 | (oil) |
| n-C₁₂H₂₅ (BDD-BBPh) | 91 | 33 - 35 |
| n-C₁₄H₂₉ (BTD-BBPh) | 94 | 33 - 35 |

GC Performance of Biphenol Ethers
FID (flame ionization detector) was used.
GC Parameter Comparison:

| Parameters | Column | |
|---|---|---|
| Column | Varian VF1701 | Agilent DB 35 |
| Maximum Temp (C) | 300 | 360 |
| Length (m) | 30 | 15 |
| Flow Rate (ml/min) | 0.9 | 1.5 |
| Initial Temp (C) | 100 | 100 |
| Hold (min) | 3 | 0 |
| Ratel (C/min) | 10 | 20 |
| Final Templ (C) | 290 | 280 |
| Hold (min) | 20 | 10 |
| Rate 2 (C/min) | | 20 |
| Final Temp2 (C) | | 340 |

| Compound | Column | |
|---|---|---|
| | Varian VF1701 | Agilent DB 35 |
| | Retention Time, Min. | |
| BOct-BBPh | 9.3 | 22.1 |
| BDec-BBPh | 11.6 | 25.8 |
| BDD-BBPh | 16.8 | 33.8 |

## Claims

1. A compound having formula (I), 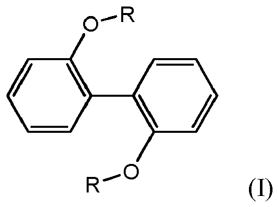 wherein R represents C₁₀-C₁₈ alkyl or C₅-C₁₈ alkenyl; wherein said alkyl group is a linear or branched hydrocarbyl group that is optionally substituted with one or more hydroxy or alkoxy groups; and wherein said alkenyl group is a linear or branched hydrocarbyl group containing one or more double bonds, and is optionally substituted with one or more hydroxy or alkoxy groups.

2. The compound of claim 1 in which R represents C₁₀-C₁₈ alkyl.

3. The compound of claim 2 in which R represents C₁₀-C₁₆ alkyl.

4. The compound of any of the preceding claims in which said alkyl and alkenyl groups are unsubstituted.

5. The compound of any of the preceding claims in which said alkyl and alkenyl groups are acyclic.

## Patentansprüche

1. Eine Verbindung mit der Formel (I): 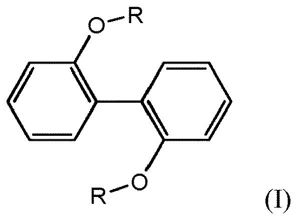 wobei R C₁₀-C₁₈-Alkyl oder C₅-C₁₈-Alkenyl darstellt; wobei die Alkylgruppe eine lineare oder verzweigte Kohlenwasserstoffgruppe ist, die optional mit einer oder mehreren Hydroxy- oder Alkoxygruppen substituiert ist; und wobei die Alkenylgruppe eine lineare oder verzweigte Kohlenwasserstoffgruppe ist, die eine oder mehrere Doppelbindungen enthält, und optional mit einer oder mehreren Hydroxy- oder Alkoxygruppen substituiert ist.

2. Verbindung gemäß Anspruch 1, bei der R C₁₀-C₁₈-Alkyl darstellt.

3. Verbindung gemäß Anspruch 2, bei der R C₁₀-C₁₆-Alkyl darstellt.

4. Verbindung gemäß einem der vorhergehenden Ansprüche, bei der die Alkyl- und Alkenylgruppen unsubstituiert sind.

5. Verbindung gemäß einem der vorhergehenden Ansprüche, bei der die Alkyl- und Alkenylgruppen azyklisch sind.

## Revendications

1. Un composé ayant la formule (I), 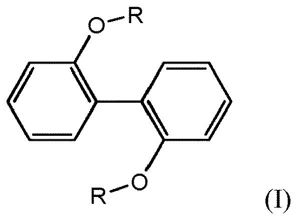 R représentant un alkyle en C₁₀-C₁₈ ou un alcényle en C₅-C₁₈ ; ledit groupe alkyle étant un groupe hydrocarbyle linéaire ou ramifié qui est facultativement substitué par un ou plusieurs groupes hydroxy ou alcoxy ; et ledit groupe alcényle étant un groupe hydrocarbyle linéaire ou ramifié contenant une ou plusieurs liaisons doubles, et étant facultativement substitué par un ou plusieurs groupes hydroxy ou alcoxy.

2. Le composé de la revendication 1 dans lequel R représente un alkyle en C₁₀-C₁₈.

3. Le composé de la revendication 2 dans lequel R représente un alkyle en C₁₀-C₁₆.

4. Le composé de n'importe lesquelles des revendications précédentes dans lequel lesdits groupes alkyle et alcényle sont non substitués.

5. Le composé de n'importe lesquelles des revendications précédentes dans lequel lesdits groupes alkyle et alcényle sont acycliques.
